# EUROPÄISCHE PATENTSCHRIFT

(11) **EP 1 114 625 B1**
(45) Veröffentlichungstag und Bekanntmachung des Hinweises auf die Patenterteilung: **11.10.2006**
(21) Anmeldenummer: 00811085.0
(22) Anmeldetag: 16.11.2000
(51) Int. Cl.: A61F 2/44, A61F 2/30

(54) **Intervertebrales Implantat**
Intervertebral implant
Implant intervertébral

(30) Priorität: 15.12.1999 EP 99811163
(43) Veröffentlichungstag der Anmeldung: 11.07.2001
(73) Patentinhaber: Zimmer GmbH, 8404 Winterthur (CH)
(72) Erfinder: Studer, Armin, 8400 Winterthur (CH); Schaffner, Silvio, 8267 Berlingen (CH); Vodermayer, Albert Maria, 8305 Dietlikon (CH); Grimberg, Gudrun, 8404 Winterthur (CH); Kärger, Jens Christian, 8405 Winterthur (CH); Erlach, Hans, 8405 Winterthur (CH)
(74) Vertreter: Manitz, Finsterwald & Partner GbR

(56) Entgegenhaltungen:
- WO-A-00/51529
- US-A- 5 084 051
- US-A- 5 192 327
- US-A- 5 429 863
- US-A- 5 766 253
- US-A- 6 074 423

## Beschreibung

Die Erfindung betrifft ein intervertebrales Implantat gemäss dem Oberbegriff des unabhängigen Patentanspruchs 1.

Ein derartiges Implantat ist beispielsweise aus FR 2 760 355 bekannt.

Intervertebrale Implantate gibt es in vielen verschiedenen Ausführungsarten. Sie werden, wie ihr Name bereits sagt, zwischen zwei benachbarten Wirbeln implantiert und kommen insbesondere dann zur Anwendung, wenn die dort angeordnete Bandscheibe funktionsuntüchtig ist oder in ihrer Funktion stark beeinträchtigt ist. Eine mögliche und relativ häufig gewählte Art der Behandlung solcher Krankheitsbilder ist, die Bandscheibe oder Teile davon zu entfernen und die beiden zueinander benachbarten Wirbel, zwischen denen die lädierte Bandscheibe angeordnet ist oder war, zu verwachsen (fusionieren). Um das Verwachsen der Wirbel zu fördern, kann das Implantat mit Knochenspänen oder Knochenersatzmaterial gefüllt werden. Die Fusion der benachbarten Wirbel soll dabei derart erfolgen, dass die Wirbel während und nach dem Zusammenwachsen ihren normalen Abstand voneinander haben, als wäre die intakte Banscheibe noch vorhanden, weil sonst die Funktion der Wirbelsäule insgesamt erheblich beeinträchtigt werden kann.

Zur Erreichung der Primärstabilität in der ersten Phase nach der Implantation dient das Implantat, welches in dieser Phase die Belastungen, die auf eine intakte Bandscheibe wirken würden, aufnehmen muss, Nach einem Zeitraum von typischerweise sechs bis neun Monaten wird die Sekundärstabilität erreicht, indem der Knochen der zwei benachbarten Wirbel durch das Implantat hindurch zusammengewachsen (fusioniert) ist, sodass die beiden benachbarten Wirbel in normalem Abstand zueinander angeordnet sind, als wenn zwischen ihnen noch eine intakte Bandscheibe vorhanden wäre. Die beiden benachbarten Wirbel sind jedoch fest miteinander verbunden (fusioniert) und können die Belastungen aufnehmen.

Zur Wahrung des Abstands zweier benachbarter Wirbel voneinander während der ersten Phase nach der Implantation - also während der Phase, in welcher die Knochen der benachbarten Wirbel noch nicht miteinander verwachsen (fusioniert) sind, gibt es verschiedenartige Implantate und auch unterschiedliche Operationstechniken, die sich schon alleine durch die Art des Zugangs zur Wirbelsäule - von anterior oder von posterior herunterscheiden.

So ist beispielsweise aus der US-A-5,192,327 ein intervertebrales Implantat bekannt, welches als ovales ringartiges Element ausgebildet ist. Mehrere solcher Elemente können zusammengesetzt werden zu einer Säule, deren Längsachse nach der Implantation in etwa in Richtung der Wirbelsäule verläuft, um gegebenenfalls auch einen Wirbel ersetzen zu können. Die Stirnflächen dieses Implantats sind offen, sodass Knochen in das Implantat einwachsen kann und eine knöcherne Fusion stattfinden kann. Das Implantat kann zur Förderung der knöchernen Fusion mit Knochenspänen oder Knochenersatzmaterial gefüllt werden. Für dieses Implantat wird als Material ein mit Karbonfasern verstärktes Kunststoffmaterial vorgeschlagen, um das Fortschreiten des Zusammenwachsens der Knochen der benachbarten Wirbel innerhalb des Implantats mit Hilfe von Röntgenstrahlen überwachen zu können. Das häufig für solche Implantate verwendete Titan erlaubt keine Kontrolle des Fortgangs beim Zusammenwachsen des Knochens der beiden Wirbel innerhalb des Implantats, weil das Implantat aus Titan für Röntgenstrahlen undurchlässig ist.

Das in der genannten US-A-5,192,327 beschriebene Implantat ist vornehmlich für die Implantation bei anteriorem Zugang geeignet. Im übrigen ist es bei diesem Implantat so, dass das Implantat alleine häufig nicht die erforderliche Primärstabilität gewährleisten kann und somit zusätzlich zu dem Implantat noch weitere Fixateure bzw. Stabilisatoren erforderlich werden, die mit den Wirbeln verbunden werden müssen (z.B. mittels Pedikelschrauben), um die notwendige Primärstabilität nach der Operation zu gewährleisten und den Erfolg der Operation nicht zu gefährden. Wird dann nach einigen Monaten die Sekundärstabilität erreicht (durch Zusammenwachsen der Wirbel), so können die zusätzlichen Fixateure bzw. Stabilisatoren in einem weiteren Eingriff wieder entfernt werden.

Um die Anbringung zusätzlicher Fixateure bzw. Stabilisatoren zu vermeiden, welche die Operation aufwendiger (und auch kostenintensiver) machen und welche darüberhinaus später mittels eines weiteren Eingriffs wieder entfernt werden können, sind bereits hohlzylindrische Implantate (sogenannte "Cages") vorgeschlagen worden, beispielsweise in der US-A-5,015,247, welche mit ihrer Längsachse quer zur Richtung der Wirbelsäule zwischen benachbarte Wirbel eingeschraubt werden. Diese hohlzylindrischen Implantate sind mit einem Aussengewinde versehen, welches sich beim Einschrauben mit dem Gewinde in die beiden Wirbelknochen, zwischen denen das Implantat eingesetzt wird, einschneidet. Dadurch sind die Implantate gegen Herausgleiten gesichert. Die Implantate sind in der Wand mit Öffnungen (Löchern) versehen, sodass Knochen durch das Implantat hindurch wachsen kann, sodass es nach einigen Monaten zu einer knöchernen Verbindung (Fusion) der beiden Wirbel kommen kann.

Diese Implantate - die Cages - haben den Vorteil, dass sie die einzigen Implantate sind, die bei der Operation benötigt werden, es werden also keinerlei zusätzliche Fixateure oder Stabilisatoren benötigt. Ein weiterer Eingriff zu einem späteren Zeitpunkt zur Entfernung zusätzlicher Fixateure bzw. Stabilisatoren ist somit nicht erforderlich. Ausserdem ist bei Verwendung solcher Implantate operationstechnisch betrachtet der Zugang sowohl von anterior als auch von posterior her relativ unkompliziert.

Bisher werden solche Cages aus grob gestrahltem Titan hergestellt, weil die entsprechend poröse Titanoberfläche einerseits das Einwachsen bzw. Aufwachsen von Knochen fördert, vor allem aber auch die erforderliche Stabilität einerseits, andererseits aber auch eine gewisse Elastizität aufweist, um die auf das Implantat in der ersten Phase nach der Operation wirkenden Belastungen zuverlässig aufnehmen zu können, sodass zusätzliche Fixateure bzw. Stabilisatoren nicht erforderlich sind. Nachteilig ist, dass bei solchen Cages das Fortschreiten der knöchernen Fusion innerhalb der Cages praktisch nicht überwacht werden kann.

Es ist daher eine Aufgabe der Erfindung, ein hohlzyindrisches intervertebrales Implantat der eingangs genannten Art vorzuschlagen (einen "Cage"), welches einerseits geeignet ist, in der ersten Phase nach der Operation die auf das Implantat wirkenden Belastungen alleine aufzunehmen, gleichzeitig jedoch eine Überwachung der knöchernen Fusion durch bildgebende Verfahren (Röntgenstrahlen, MRI, CT, etc.) innerhalb des Implantats zu ermöglichen.

Diese Aufgabe wird durch das intervertebrale Implantat, wie es durch die Merkmale des unabhängigen Patentanspruchs 1 charakterisiert ist, gelöst. Besonders vorteilhafte Ausgestaltungen ergeben sich aus den Merkmalen der abhängigen Patentansprüche.

Das erfindungsgemässe Implantat weist die Vorteile auf, dass es einerseits die in der ersten Phase nach der Operation auf das Implantat wirkenden Belastungen aufnehmen kann, dass es das einzige Implantat ist und keine zusätzlichen Fixateure oder Stabilisatoren erforderlich sind, dass der Zugang sowohl von anterior als auch von posterior auf relativ einfache Weise möglich ist, und dass das Fortschreiten der knöchernen Fusion innerhalb des Implantats mit Hilfe von Röntgenuntersuchungen bzw. anderen bildgebenden Verfahren (MRI, CT, etc.) überwacht werden kann.

Der Kunststoff kann beispielsweise in faseriger Form vorliegen und zu der gewünschten Form des Implantats gewickelt und anschliessend einer Presssinterung unterzogen werden. Beim Presssintern bleibt die grundsätzliche Orientierung der Kunststoffmoleküle der Fasern erhalten, wodurch dem Implantat die Steifigkeit verliehen wird, jedoch werden die Fasern beim Presssintern so weit aufgeschmolzen, dass sie sich miteiander verbinden und einen festen Verbund in Form des Implantats bilden. Bei dem erfindungsgemässen Implantat umfasst das für Röntgenstrahlen durchlässige Material einen mit Fasermaterial verstärkten Kunststoff.

Dabei kommen verschiedene Varianten in Frage, wie im einzelnen noch erläutert werden wird. So kann man beispielsweise endlosfaserverstärkte Kunststoffe ("Tapes") wickeln und anschliessend verpressen und ggf. den so hergestellten Körper noch mit einem Kunststoff umgeben. Mit quasiendlosfaserverstärkten Materialien ("commingled stretch broken yarn") - das sind Materialien, bei denen die endlosen Fasern gebrochen, aber miteinander verwickelt sind, um dem Material eine gewisse Längselastizität zu verleihen - ist es ebenfalls möglich, zu wickeln und anschliessend zu verpressen und die so hergestellten Körper ggf. noch mit einem Kunststoff zu umgeben. Bei sogenannten langfaserverstärkten Kunststoffen (Faserlängen typischerweise im Bereich von 3 mm bis hin zu 6 mm oder sogar 10 mm) liegen die diskreten Fasern schon im Kunststoff vor und das Implantat wird z.B. durch Fliesspressen hergestellt. Schliesslich kommen noch kurzfaserverstärkte Kunststoffe in Frage (Faserlängen typischerweise im Bereich von 0.1 mm bis 0.4 mm), die insbesondere bei spritzbaren Kunststoffen im Spritzgiessverfahren zu dem Implantat verarbeitet werden können. Weitere vorteilhafte Varianten können sich aus auch der Kombination der verschiedenen Herstellungsverfahren ergeben.

Die genannten Materialien sind besonders geeignet zur Aufnahme der unmittelbar nach der Operation auf das Implantat wirkenden Lasten und sind natürlich für Röntgenstrahlen durchlässig, sodass das Fortschreiten der knöchernen Fusion innerhalb des Implantats mit Hilfe von Röntgenuntersuchungen überwacht werden kann.

Bei einer Weiterbildung des Implantats ist der der Kunststoff ein spritzbarer Kunststoff. Die Spritzgusstechnik erlaubt eine zuverlässige und gleichzeitig kostengünstige Herstellung des Implantats.

Das Fasermaterial umfasst vorteilhafterweise Karbonfasern und der Kunststoff ist vorzugsweise Polyetheretherketon (PEEK). Die Karbonfasern nehmen in zuverlässiger Weise die unmittelbar nach der Operation auftretenden Lasten auf und können mit Polyetheretherketon umspritzt werden, weil dies ein spritzbarer Kunststoff ist, der zudem auch noch biokompatibel ist.

Erfindugsgemäß umfasst das Implantat drei aus Fasermaterial hergestellte, ringförmig ausgebildete Körper, welche in Kunststoff eingebettet sind. Die Lastaufnahme erfolgt im Bereich der ringförmig ausgebildeten Körper, die unterschiedlich über die Länge des Implantats verteilt sein können.

Bei einer ersten Variante mit drei solchen ringförmig ausgebildeten Körpern ist einer der ringförmig ausgebildeten Körper im zentralen Bereich des Implantats angeordnet und die beiden anderen im jeweiligen Endbereich des Implantats. Dies erlaubt eine Belastung des Implantats sowohl im zentralen Bereich als auch in den Endbereichen.

Bei einer weiteren Variante sind alle drei ringförmigen Körper mehr oder weniger im zentralen Bereich des Implantats angeordnet. Die Endbereiche des Implantats sind jeweils mit einer Fase versehen. Die Lastaufnahme erfolgt bei dieser Variante mehr oder weniger ausschliesslich im zentralen Bereich, weshalb in diesem Bereich auch sämtliche ringförmigen Körper angeordnet sind. Dies ist deshalb so, weil der Endbereich mit einer Fase versehen ist und dort in der ersten Phase nach der Operation keinen lastaufnehmenden Kontakt mit dem Knochen hat. Die Lastaufnahme soll bei diesem Ausführungsbeispiel also gezielt in dem zentralen Bereich erfolgen. Die Fase in den Endbereichen erleichtert das Eindrehen des Implantats während der Operation.

Schlieslich kann bei sämtlichen zuvor genannten Ausführungsbeispielen die Oberfläche des Implantats mit einer die Osteointegration fördernden Schicht, insbesondere mit einer Hydroxylapatitkeramikschicht, versehen sein.

Im folgenden wird die Erfindung anhand der Zeichnung näher erläutert. Es zeigen, zum Teil stark vergrössert und schematisch bzw. im Schnitt:
- Fig. 1: ein erstes Ausführungsbeispiel eines erfindungsgemässen intervertebralen Implantats mit drei ringförmigen Körpern aus Fasermaterial, einer im zentralen Bereich und die beiden anderen im jeweiligen Endbereich,
- Fig. 2: ein zweites Ausführungsbeispiel eines erfindungsgemässen Implantats mit drei ringförmigen Körpern aus Fasermaterial, die jedoch alle im zentralen Bereich des Implantats angeordnet sind, und
- Fig. 3: ein drittes Ausführungsbeispiel eines erfindungsgemässen Implantats mit drei ringförmigen Körpern aus Fasermaterial, einer im zentralen Bereich und die beiden anderen im jeweiligen Endbereich, wobei der Körper im zentralen Bereich einen rechteckigen und die beiden Körper in den Endbereichen einen kreisförmigen Querschnitt aufweisen.

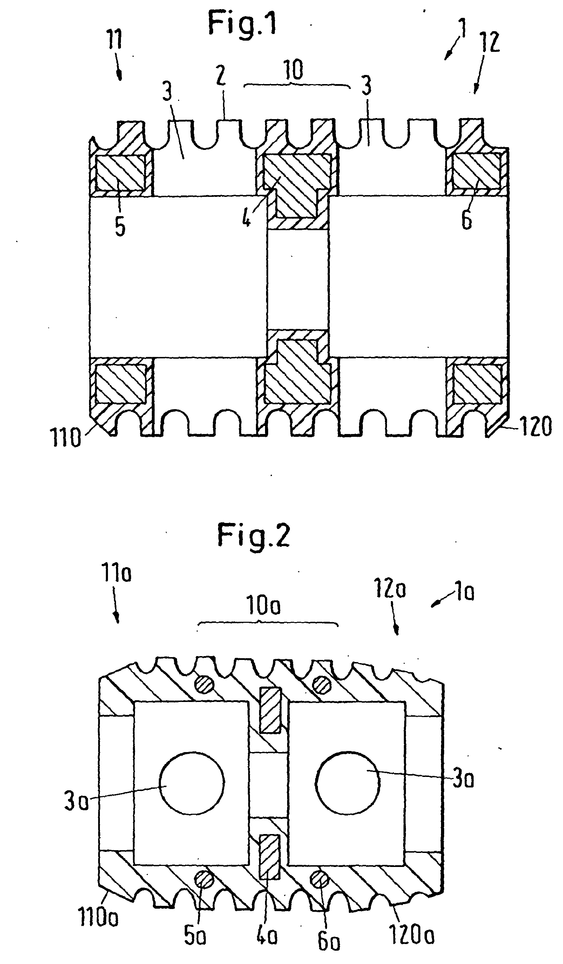

In Fig. 1 erkennt man ein erstes Ausführungsbeispiel eines erfindungsgemässen intervertebralen Implantats 1, welches mit einem Aussengewinde 2 versehen ist, damit das Implantat 1 zwischen zwei Wirbel (nicht dargestellt) eingeschraubt werden kann. Das Implantat 1 weist eine hohlzylindrische Gestalt auf und ist in seiner Wand mit Öffnungen 3 versehen, durch welche hindurch Knochen in das Implantat 1 hinein einwachsen kann. Das Implantat 1 weist drei ringförmige Körper 4,5,6 aus Fasermaterial auf, von denen der Körper 4 im zentralen Bereich 10 und die beiden Körper 5 und 6 in den beiden Endbereichen 11 und 12 des Implantats 1 angeordnet sind. Die ringförmigen Körper 4,5,6 sind von einem Kunststoff umgeben. In den Endbereichen erkennt man ein ganz leichte Fase 110 bzw. 120, welche dem leichteren Einschrauben des Implantats 1 dienen. Der ringförmige Körper 4 im zentralen Bereich 10 des Implantats 1 ist etwas stärker ausgeführt als die beiden ringförmigen Körper 5 und 6 in den Endbereichen 11 und 12 des Implantats 1, weil die grössere Belastung des Implantats 1 im zentralen Bereich 10 erfolgt. Das in Fig. 1 gezeigte Ausführungsbeispiel ist sowohl im zentralen Bereich 10 als auch in den beiden Endbereichen 11 und 12 belastbar und steht dort auch mit dem umgebenden Knochenmaterial in lastaufnehmender Weise in Kontakt.

Das Implantat 1 lässt sich beispielsweise derart herstellen, dass zunächst die ringförmigen Körper 4,5,6 aus einem endlosfaserverstärkten Kunststoff als Fasermaterial gewickelt, danach einer Presssinterung unterzogen werden und dann in einer definierten Position in eine Spritzgussform eingebracht werden, wo die pressgesinterten ringförmigen Körper 4,5,6 mit Kunststoff (z.B. PEEK) umspritzt werden können. Auf diese Weise entsteht ein Implantat 1, bei welchem das Fortschreiten der knöchernen Fusion mit Hilfe von Röntgenstrahlen gut überwacht werden kann.

Bei dem zweiten Ausführungsbeispiel gemäss Fig. 2 umfasst das Implantat 1a ebenfalls drei ringförmige Körper 4a,5a,6a aus Fasermaterial (z.B. aus einem endlosfaserverstärkter Kunststoff), wobei aber alle drei ringförmigen Körper 4a,5a,6a mehr oder weniger im zentralen Bereich 10a des Implantats 1a angeordnet sind. Die Endbereiche 11 a und 12a sind jeweils mit einer deutlich ausgeprägten Fase 110a bzw. 120a versehen. Diese Fasen 110a bzw. 120a dienen zur erheblichen Erleichterung beim Einschrauben des Implantats 1a, allerdings stehen sie nach der Operation nicht in lastaufnehmendem Kontakt mit dem Knochen. Das Implantat 1a muss also mehr oder weniger die gesamte Last im zentralen Bereich 10a aufnehmen, weshalb das Implantat dort auch mit drei ringförmigen Körpern 4a,5a,6a versehen ist. Ausserdem erkennt man in Fig.2 noch die Öffnungen 3a, durch welche hindurch der Knochen in das Implantat 1a hinein einwachsen kann. Die Herstellung kann analog erfolgen wie bei dem Ausführungsbeispiel gemäss Fig. 1, wobei jedoch nach dem ersten Spritzvorgang die Öffnungen 3a hergestellt werden (z.B. durch Bohren bzw. Fräsen) und dann ggf. ein weiterer Spritzvorgang erfolgt, damit nicht eventuell Teile des Fasermaterials in den Körper gelangen können.

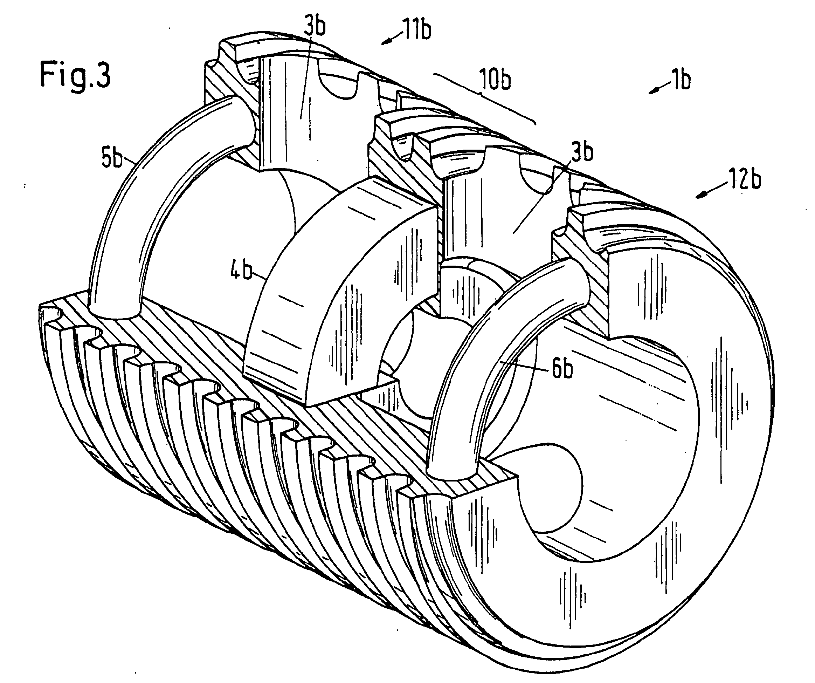

In Fig. 3 ist ein drittes Ausführungsbeispiel eines erfindungsgemässen Implantats 1b dargestellt. Bei diesem Implantat 1b sind wieder drei ringförmige Körper 4b,5b,6b aus Fasermaterial (z.B. aus einem endlosfaserverstärkten Kunststoff) zu erkennen, wobei der Körper 4b im zentralen Bereich 10b und die beiden anderen Körper 5b,6b jeweils im Endbereich 11 b bzw. 12b angeordnet sind. Ferner erkennt man die Öffnungen 3b, durch welche hindurch der Knochen in das Implantat 1b hinein einwachsen kann, sodass es zu einer Fusion kommen kann. Der Querschnitt der ringförmigen Körper kann jeweils den übrigen Gegebenheiten angepasst werden, insbesondere der gewünschten Lastaufnahme bzw. dem Raum, der zum Umspritzen mit Kunststoff (z.B. PEEK) zur Verfügung steht. Bei dem in Fig. 3 gezeigten Ausführungsbeispiel weist der im zentralen Bereich 10b angeordnete ringförmige Körper 4b einen rechteckigen Querschnitt auf, während die in den Endbereichen angeordneten Körper 5b und 6b einen kreisförmigen Querschnitt aufweisen. Die Herstellung kann analog erfolgen wie bei dem Ausführungsbeispiel gemäss Fig. 1.

Was die Materialien für sämtliche beschriebenen Ausführungsbeispiele betrifft, so kommen als Fasern für das Fasermaterial insbesondere die nachfolgenden Fasern in Frage: Organische Fasern wie z.B. Aramid- und PBO-Fasern (Poly(p-phenylen-2,6,-benzobisoxazole), sowie anorganische Fasern wie z.B. Karbonfasern, Aluminiumoxidfasern, Zirkonoxidfasern und Borfasern. Das Fasermaterial sind dann vorzugsweise thermoplastische Matrices aus solchen Fasern und einem thermoplastischen Kunststoff, wie z.B. das bereits früher genannte PEEK (Polyetheretherketon), aber auch Kunststoffe wie PES (Polyethersulfon), PSU (Polysulfon), PET (Polyethylenterephthalat), UHMWPE (Ultrahochmolekulargewichtiges Polyethylen), PEI (Polyetherimid). Möglich ist auch eine duroplastische Matrix aus EP (Epoxidharz).

Ebenfalls ist es denkbar, dass das Implantat nicht mit einem wie auch immer gewickelten Körper aus Fasermaterial versehen ist, sondern dass die Fasern als geschnittene bzw. gebrochen Fasern in dem Kunststoff eingelagert sind (kurzfaserverstärkte Kunststoffe, langfaserverstärkte Kunststoffe, "commingled strecht broken yarn", siehe weiter vorne) und das Implantat aus einem solchen mit Fasermaterial verstärkten Kunststoff als Ausgangsstoff hergestellt wird, der in einem der genannten Kunststoffe eingebettet ist. Wie bereits früher erwähnt, kann die Oberfläche des Implantats noch mit einer die Osteointegration des Implantats fördernden dünnwandigen Schicht, insbesondere einer Hydroxylapatitkeramikschicht, versehen sein.

## Patentansprüche

1. Intervertebrales Implantat (1) mit einer hohlzylindrischen Gestalt, welches Implantat mit einem Aussengewinde (2) versehen ist zum Einschrauben des Implantats in den Zwischenraum zwischen zwei benachbarten Wirbeln, und welches in seiner Wand mit Öffnungen (3) versehen ist, durch welche hindurch Knochen durch das Implantat (1) hindurch wachsen kann, wobei das Implantat (1) aus einem für Röntgenstrahlen durchlässigen Material hergestellt ist,
**dadurch gekennzeichnet, dass** das für Röntgenstrahlen durchlässige Material einen mit Fasermaterial verstärkten Kunststoff umfasst, und dass das Implantat (1,1a,1b) drei aus Fasermaterial hergestellte, ringförmig ausgebildete Körper (4,5,6;4a,5a,6a;4b,5b,6b) umfasst, welche in Kunststoff eingebettet sind.

2. Intervertebrales Implantat nach Anspruch 1,
**dadurch gekennzeichnet, dass** das für Röntgenstrahlen durchlässige Material einen Kunststoff umfasst oder aus einem Kunststoff hergestellt ist.

3. Intervertebrales Implantat nach Anspruch 1 oder 2,
**dadurch gekennzeichnet, dass** der Kunststoff ein spritzbarer Kunststoff ist.

4. Intervertebrales Implantat nach einem der Ansprüche 1 bis 3,
**dadurch gekennzeichnet, dass** das Fasermaterial Karbonfasern umfasst und der Kunststoff Polyetheretherketon (PEEK) ist.

5. Intervertebrales Implantat (1,1b) nach einem der vorhengehenden der Anspruche,
**dadurch gekennzeichnet, dass** einer der ringförmig ausgebildeten Körper (4,4b) im zentralen Bereich (10,10b) des Implantats angeordnet ist und die beiden anderen (5,6;5b,6b) im jeweiligen Endbereich (11,12;11b,12b) des Implantats.

6. Intervertebrales Implantat (1a) nach einem der Ansprüche 1 bis 4,
**dadurch gekennzeichnet, dass** alle drei ringförmigen Körper (4a,5a,6a) im zentralen Bereich (10a) des Implantats (1a) angeordnet sind und bei welchem die Endbereiche (11a,12a) des Implantats (1a) mit einer Fase (110a,120a) versehen sind.

7. Intervertebrales Implantat nach einem der vorangehenden Ansprüche,
**dadurch gekennzeichnet, dass** die Oberfläche des Implantats mit einem die Osteointegration fördernden Material, insbesondere mit Hydroxylapatitkeramik, versehen ist.

## Claims

1. Intervertebral implant (1) with a hollow cylindrical shape, said implant being provided with an external thread (2) for screwing the implant into the intermediate space between two adjacent vertebrae and being provided with openings (3) in its wall through which bone can grow through the implant (1), with the implant (1) being manufactured of a material permeable for X-rays,
**characterized in that**
the material permeable for X-rays comprises a plastic reinforced by a fiber material; and
**in that** the implant (1, 1a, 1b) comprises three bodies (4, 5, 6; 4a, 5a, 6a; 4b, 5b, 6b) which are manufactured of fiber material, are formed in ring shape and are embedded in plastic.

2. Intervertebral implant in accordance with claim 1, **characterized in that** the material permeable for X-rays comprises a plastic or is manufactured of a plastic.

3. Intervertebral implant in accordance with claim 1 or claim 2, **characterized in that** the plastic is an injection moldable plastic.

4. Intervertebral implant in accordance with any one of the claims 1 to 3, **characterized in that** the fiber material comprises carbon fibers and the plastic is polyether ether ketone (PEEK).

5. Intervertebral implant (1, 1b) in accordance with any one of the preceding claims, **characterized in that** one of the bodies (4a, 4b) formed in ring shape is arranged in the central region (10, 10b) of the implant and the other two (5, 6; 5b, 6b) are arranged in the respective end region (11, 12; 11b, 12b) of the implant.

6. Intervertebral implant (1a) in accordance with any one of the claims 1 to 4, **characterized in that** all three ring-shaped bodies (4a, 5a, 6a) are arranged in the central region (10a) of the implant (1a); and **in that** the end regions (11a, 12a) of the implant (1a) are provided with a chamfer (110a, 120a).

7. Intervertebral implant in accordance with any one of the preceding claims, **characterized in that** the surface of the implant is provided with a material promoting osteo-integration, in particular comprising hydroxylapatite ceramic.

## Revendications

1. Implant intervertébral (1) présentant une configuration cylindrique creuse, ledit implant étant pourvu d'un filetage (2) pour visser l'implant dans l'intervalle entre deux vertèbres voisines, et étant pourvu d'ouvertures (3) dans sa paroi, à travers lesquelles la substance osseuse peut croître à travers l'implant (1), l'implant (1) étant réalisé à partir d'un matériau perméable aux rayons X, **caractérisé en ce que** le matériau perméable aux rayons X contient une matière plastique renforcée de fibres, et **en ce que** l'implant (1, 1a, 1b) comprend trois corps (4, 5, 6, ; 4a, 5a, 6a ; 4b, 5b, 6b) réalisés en forme annulaire à partir d'un matériau de fibres et noyés dans une matière plastique.

2. Implant intervertébral selon la revendication 1, **caractérisé en ce que** le matériau perméable aux rayons X contient une matière plastique ou est réalisé en matière plastique.

3. Implant intervertébral selon l'une ou l'autre des revendications 1 et 2, **caractérisé en ce que** la matière plastique est une matière plastique injectable.

4. Implant intervertébral selon l'une des revendications 1 à 3, **caractérisé en ce que** le matériau de fibres contient des fibres de carbone et la matière plastique est du polyétheréthercétone (PEEK).

5. Implant intervertébral (1, 1b) selon l'une des revendications précédentes, **caractérisé en ce que** l'un des corps réalisés annulaires (4, 4b) est agencé dans la zone centrale (10, 10b) de l'implant et les deux autres corps (5, 6 ; 5b, 6b) sont agencés dans la zone d'extrémité respective (11, 12 ; 11 b, 12b) de l'implant.

6. Implant intervertébral (1a) selon l'une des revendications 1 à 4, **caractérisé en ce que** tous les trois corps annulaires (4a, 5a, 6a) sont agencés dans la zone centrale (10a) de l'implant (1a), et **en ce que** les zones d'extrémité (11a, 12a) de l'implant (1a) sont pourvues d'un chanfrein (110a, 120a).

7. Implant intervertébral selon l'une des revendications précédentes, **caractérisé en ce que** la surface de l'implant est pourvue d'un matériau favorisant l'ostéo-intégration, en particulier de céramique d'hydroxylapatite.
